(19) Europäisches Patentamt European Patent Office Office européen des brevets

(11) **EP 4 549 932 A1**

(12) **EUROPEAN PATENT APPLICATION**

(43) Date of publication:
**07.05.2025  Bulletin 2025/19**

(21) Application number: **23207597.8**

(22) Date of filing: **03.11.2023**

(51) International Patent Classification (IPC):
**G01N 33/44** (2006.01)     **G01N 21/84** (2006.01)
**G01N 21/64** (2006.01)

(52) Cooperative Patent Classification (CPC):
**G01N 21/6408**; G01N 33/442; G01N 2021/845

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Technische Hochschule Rosenheim, in Vertretung des Freistaates Bayern**
**83024 Rosenheim (DE)**

(72) Inventors:
• **Versen, Martin**
  **83620 Feldkirchen-Westerham (DE)**

• **Leiter, Nina**
  **83626 Valley (DE)**
• **Wohlschläger, Maximilian**
  **83714 Miesbach (DE)**
• **Karlinger, Peter**
  **83024 Rosenheim (DE)**
• **Zillmer, Markus**
  **83026 Rosenheim (DE)**

(74) Representative: **Koplin, Moritz**
  **Anne-Conway-Straße 1**
  **28359 Bremen (DE)**

(54) **WATER CONTENT DETERMINATION**

(57)     Provided is a method and a system. The method comprises exposing one or more samples to electromagnetic radiation and determining a parameter which is indicative of a water content of the one or more samples by analyzing one or more fluorescence properties of the one or more samples. The system comprises an apparatus for manufacturing a product from plastic and an inline monitoring device which determines a parameter that is indicative of a water content of the plastic by analyzing one or more fluorescence properties of the plastic.

**Fig. 12**

EP 4 549 932 A1

## Description

FIELD

The present disclosure relates to the determination of a parameter which is indicative of a water content of a sample. More particularly, the present disclosure relates to the determination of a parameter which is indicative of a water content of, and in particular traces of water in, solid plastic.

BACKGROUND

The water content of plastic may influence the quality of products made thereof. However, known processes for determining the water content of organic samples such as the Karl Fischer titration cause the destruction of the samples and are relatively slow. Another disadvantage of this method is that it cannot be integrated into a running manufacturing process of plastic components due to its lack of real-time capability.

SUMMARY

The present invention is based on the finding that the water content of a sample has an influence on the sample's fluorescent behavior and, in particular, on the fluorescence lifetime. As a consequence, the water content of a sample can be determined based on the fluorescence lifetime which allows for a nondestructive and relatively fast determination of the water content which may be suitable for inline monitoring of industrial processes such as manufacturing.

A method according to the present invention comprises exposing one or more samples to electromagnetic radiation and determining a parameter which is indicative of a water content of the one or more samples by analyzing one or more fluorescence properties of the one or more samples.

In this regard, the term "sample", as used throughout the description and the claims, particularly refers to organic matter. Moreover, the term "organic matter", as used throughout the description and the claims, particularly refers to matter formed by compounds containing carbons such as compounds containing carbon-hydrogen or carbon-carbon bonds.

Furthermore, the formulation "a parameter which is indicative of a water content of the one or more samples", as used throughout the description and the claims, particularly refers to a parameter which expresses the volumetric or gravimetric water content or from which the volumetric or gravimetric water content can be calculated.

Moreover, the formulation "analyzing one or more fluorescence properties", as used throughout the description and the claims, particularly refers to assigning one or more numeric values which are indicative of the one or more fluorescence properties to a numeric representation of the parameter. For example, the one or more numeric values may be arguments of a reference function which allows calculating the numeric representation of the parameter.

The one or more fluorescence properties may include a fluorescence lifetime or a measure which is indicative of the fluorescence lifetime. The fluorescence lifetime or a measure which is indicative of the fluorescence lifetime may be determined by analyzing sensor data representing properties of the electromagnetic radiation emitted by the one or more samples in the frequency domain. Analyzing the sensor data in the frequency domain may involve frequency domain fluorescence lifetime imaging microscopy.

The one or more fluorescence properties may include a fluorescence intensity or a measure which is indicative of the fluorescence intensity. For instance, increasing the water content of an organic sample may cause the fluorescence intensity of the organic sample to decrease as the water absorbs electromagnetic radiation. Thus, a fluorescence intensity which is lower than a reference value derived from a reference sample (e.g., by Karl Fischer titration) may indicate a higher water content than the reference sample.

Moreover, the water may react with one or more of the chromophores which exhibit fluorescence. Thus, a fluorescence intensity which is higher or lower than a reference value derived from a reference sample (e.g., by Karl Fischer titration) may indicate a water content that differs from the water content of the reference sample.

The one or more samples may comprise organic matter.

The one or more samples may comprise autofluorescent organic matter.

The one or more samples may comprise plastic.

The one or more samples may comprise autofluorescent plastic.

The method may further comprise controlling a manufacturing process which involves processing the one or more samples, based on the parameter.

For example, the manufacturing process may be adapted to ensure the quality of a product made by processing the one or more samples. Moreover, the parameter may be stored for documentation purposes. Furthermore, the parameter may be used to monitor the manufacturing process. For instance, an alarm may be raised if the parameter is found to be outside a reference range.

Controlling the manufacturing process based on the parameter may involve increasing or decreasing a water content of a raw material which comprises the one or more samples if the parameter is outside a reference range.

For example, the raw material from which the one or more samples were taken may be dried or humidified depending on whether the water content was determined as being too high or too low.

Controlling the manufacturing process based on the parameter may involve identifying a sample or a product which comprises the one or more samples as non-conforming if the parameter is outside a reference range.

The non-conforming sample or product may then be separated from conforming samples or products and depending on the severity of the non-conformity, repaired, used for other purposes, recycled, or discarded.

The method may further comprise collecting information which is indicative of a water content of different parts of one of the one or more samples by analyzing the one or more fluorescence properties of the different parts.

For example, an apparatus for sensing the fluorescence may comprise a multitude of sensors, wherein each sensor captures electromagnetic radiation emitted by a different part of a sample. This may allow creating a two-dimensional or three-dimensional representation of a sample which indicates the water content distribution across the sample. Moreover, the sample may be inspected under different angles to improve the accuracy and/or completeness of the two-dimensional or three-dimensional representation.

The method may further comprise generating a two-dimensional or three-dimensional representation of said one of the one or more samples, wherein the representation indicates a water content distribution across said one of the one or more samples and comparing the representation to a model or a reference.

The model may be based on a precomputed (desired or acceptable) water content distribution.

A system according to the present invention comprises an apparatus for manufacturing a product from plastic and an inline monitoring device which determines a parameter that is indicative of a water content of the plastic by analyzing one or more fluorescence properties of the plastic.

The plastic may be autofluorescent plastic.

The inline monitoring device may be configured to measure a fluorescence lifetime of the plastic in a frequency domain.

For example, the inline monitoring device may be configured to perform frequency domain fluorescence lifetime imaging microscopy. Performing frequency domain fluorescence lifetime imaging microscopy may involve modulating the amplitude of the electromagnetic radiation used for exciting fluorescence and measuring the phase shift between the incident and the emitted electromagnetic radiation.

The system may further comprise a camera, wherein the inline monitoring device is configured to record image data of the plastic as the plastic passes through an area onto which the camera is focused.

The system may be configured to switch a supply or to modify a pretreatment of the plastic if the parameter is outside a reference range.

The system may further comprise another inline monitoring device which determines another parameter that is indicative of a water content of the product by analyzing one or more fluorescence properties of the product.

Thus, the system may be configured to test both, the raw material from which the product is manufactured as well as the manufactured product.

Notably, the features of the system may be features of the method and vice versa.

Moreover, the invention also extends to the inline monitoring device as a standalone component of the system.

Furthermore, the inline monitoring device may not only be used for inline monitoring, but more general as a device for determining a parameter which is indicative of a water content of one or more samples (e.g., samples containing or being comprised of fluorescent organic matter) by analyzing one or more fluorescence properties of the one or more samples. For example, the device may be used for controlling a manufacturing process based on the monitored parameter.

BRIEF DESCRIPTION OF THE DRAWINGS

The foregoing aspects and many of the attendant advantages will become more readily appreciated as the same become better understood by reference to the following description of embodiments, when taken in conjunction with the accompanying drawings, wherein like reference numerals refer to like parts throughout the various views, unless otherwise specified.

Fig. 1 schematically illustrates an inline monitoring device which is configured to determine a parameter that is indicative of a water content of a sample which is at rest;

Fig. 2 schematically illustrates an inline monitoring device which is configured to determine a parameter that is indicative of a water content of a sample which is moving;

Fig. 3 schematically illustrates how the inline monitoring device of Fig. 2 may be used to separate out non-conforming samples;

Fig. 4 schematically illustrates how the inline monitoring device of Fig. 2 may be used to condition non-conforming samples (products);

Fig. 5 schematically illustrates how the inline monitoring device of Fig. 2 may be used to condition non-conforming samples (raw material);

Fig. 6 schematically illustrates how the inline monitoring device of Fig. 2 may be used to mix raw materials to ensure sample (product) conformity;

Fig. 7 schematically illustrates how the inline monitoring device of Fig. 2 may be used to ensure sample (raw material) conformity;

Fig. 8 schematically illustrates how the approaches employed in Fig. 6 and Fig. 7 may be combined;

Fig. 9 schematically illustrates how the approaches employed in Fig. 4 and Fig. 7 may be combined;

Fig. 10 schematically illustrates another example of integrating the inline monitoring device into a manufacturing system;

Fig. 11 schematically illustrates yet another example of integrating the inline monitoring device into a manufacturing system;

Fig. 12 schematically illustrates a manufacturing system for plastic casting in which the inline monitoring device may be used for controlling the casting process;

Fig. 13 shows a flow-chart of the steps performed by the inline monitoring device;

Fig. 14 schematically illustrates how the fluorescence lifetime may be measured in the frequency domain; and

Fig. 15 schematically illustrates how fluorescence properties may be measured across different parts of a sample.

Notably, the drawings are not drawn to scale and unless otherwise indicated, they are merely intended to conceptually illustrate the structures and procedures described herein.

DESCRIPTION OF EMBODIMENTS

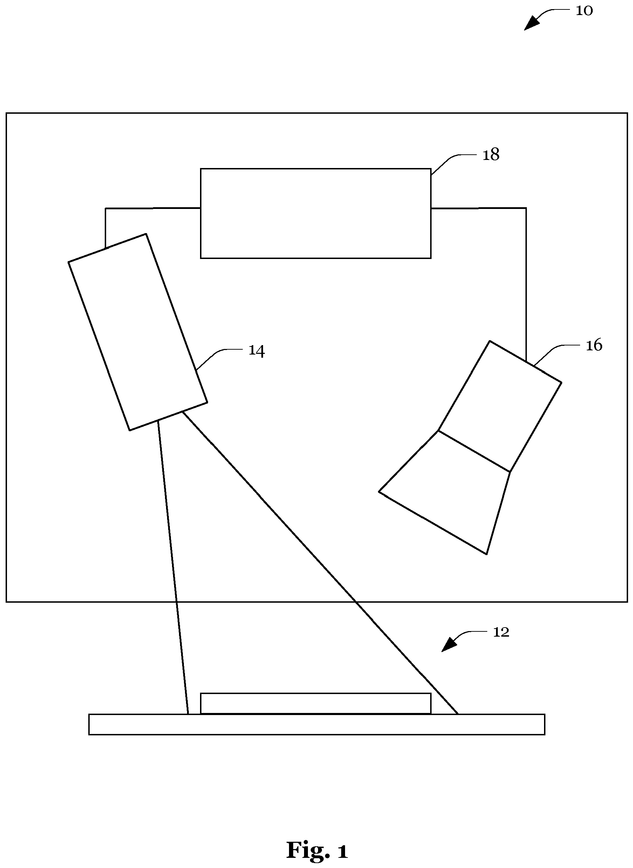

Fig. 1 shows a device 10 for determining a parameter that is indicative of a water content of a sample 12. The device 10 comprises an emitter 14, a detector 16, and a controller 18. The emitter 14 is configured to emit electromagnetic radiation within a predetermined frequency range onto the sample 12. The sample 12 contains fluorescent chromophores which absorb the electromagnetic radiation and reemit electromagnetic radiation which is then captured by the detector 16. The detector 16 may comprise a multitude of optical sensors, wherein each sensor detects electromagnetic radiation from a certain part of the sample 12.

The controller 18 which provides control over the operation of the emitter 14 and the detector 16, receives the measurement data from the detector 16 and analyzes the fluorescence properties of the sample 12. For example, the controller 18 may analyze the fluorescence lifetime and compare the measured fluorescence lifetime with an expected fluorescence lifetime. The expected fluorescence lifetime may be based on calculations pertaining to a model, measurements involving a reference sample, or measurements of one or more samples 12 (within the same batch) which have already been processed by the device 10. For instance, the device 10 may check all samples 12 from a batch and indicate (short-term or long-term) changes regarding the determined water content.

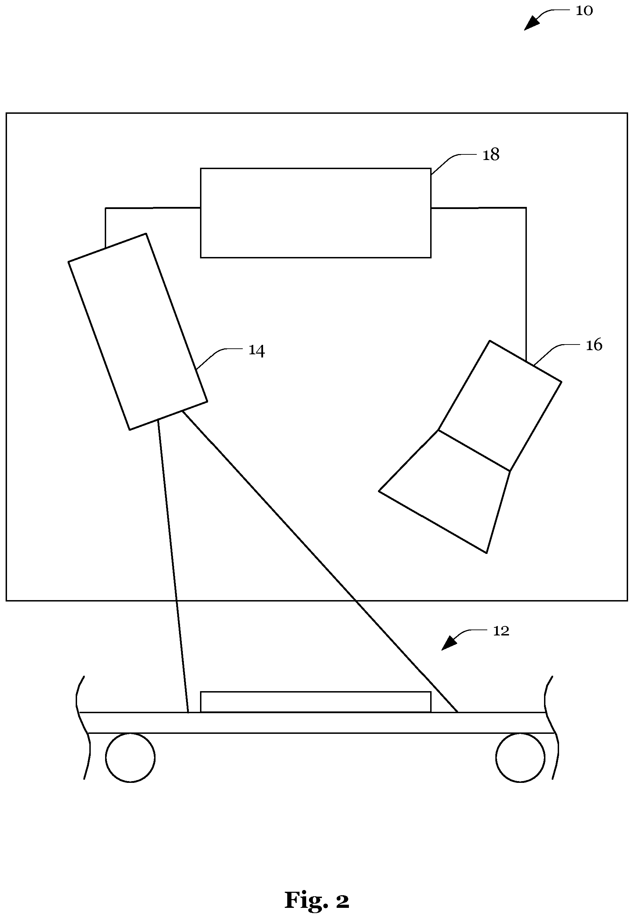

Whereas measurements may be performed while the sample 12 is at rest as illustrated in Fig. 1, measurements may also be performed while the sample 12 is in motion as shown in Fig. 2 where the sample 12 is transported on a conveyor belt past the detector 16 as the detector 16 measures the electromagnetic radiation emitted by the sample 12. If the sample 12 is moving at high speed, the controller 18 may take the movement into account by assigning measurement data from a single sensor to different parts of the sample 12 as each of said parts moves through the region monitored by the sensor.

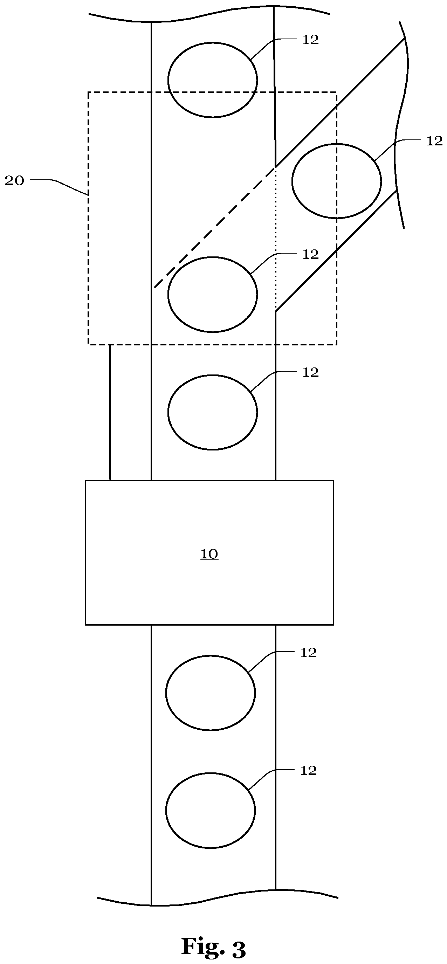

As indicated in Fig. 3, a junction 20 which may be controlled by the controller 18 may be installed downstream of the device 10 to separate non-conforming samples 12 from conforming samples 12. For example, a sample 12 may be conforming if its water content is within a predetermined range or when the water content of one or more specific parts of the sample 12 is within a predetermined range.

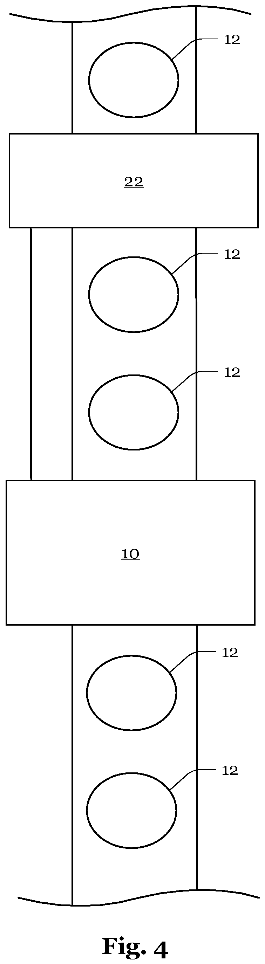

As indicated in Fig. 4, non-conforming samples 12 may be made conforming by a conditioner 22 which may be controlled by the controller 18. If a sample 12 is (part of) a final product, the conditioner 22 may remove or add water to the sample 12 as required to make the sample 12 conforming. Alternatively, or in addition, the conditioner 22 may take further measures to overcome adverse effects that would otherwise result from the non-conforming water content of the sample 12. For example, if the determined water content of the sample 12 indicates a structural weakness of the sample 12, measures may be taken to overcome or compensate the structural weakness of the sample 12 despite the non-conforming water content.

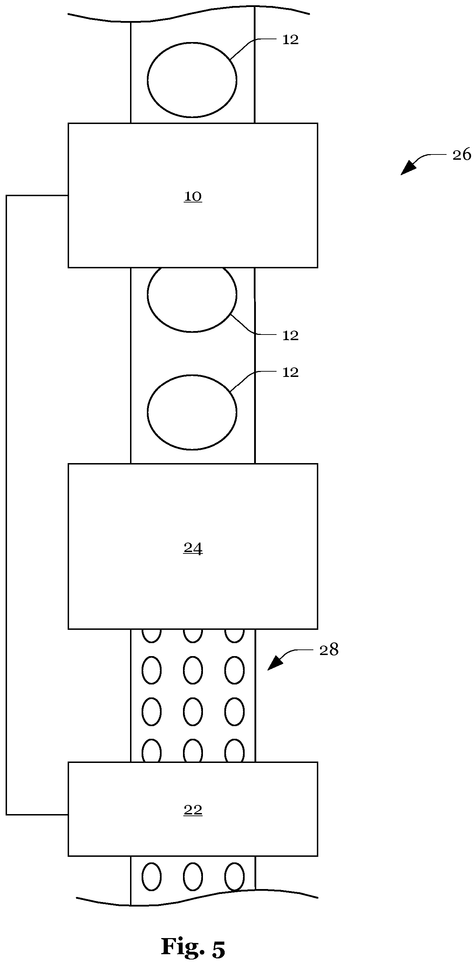

As shown in Fig. 5, the conditioner 22 may also be arranged in a manufacturing system 26 upstream of an apparatus 24 which manufactures the samples 12. Hence, the conditioner 22 may treat raw materials 28 which are processed by the apparatus 24 in a way that causes the water content of the samples 12 to be conforming. For example, the samples 12 may be products manufactured from plastic raw material and water may be removed or added to the plastic raw material to ensure a conforming water content of the samples 12.

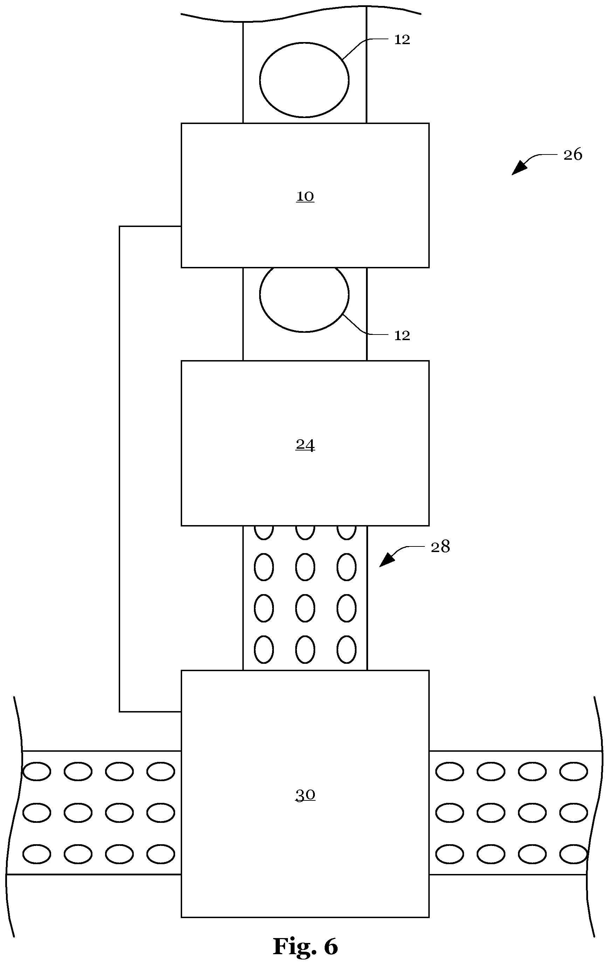

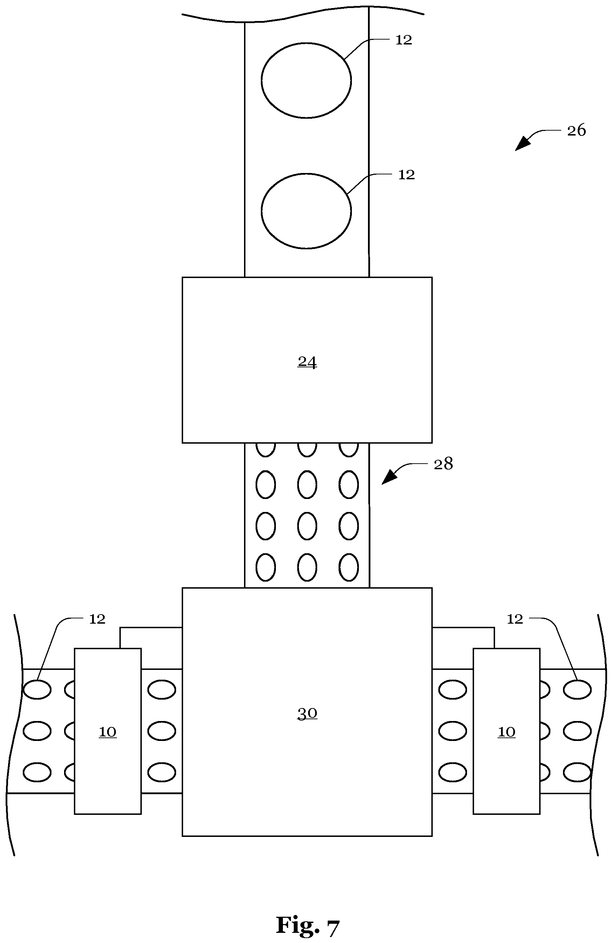

As shown in Fig. 6, the conditioner 22 may be replaced by a mixer 30 which is fed with raw materials from different supplies, and which is configured to mix the raw materials at a ratio which ensures a conforming water content of the samples 12. Instead of mixing raw materials from different supplies, the system 26 may also switch between different supplies. Moreover, as shown in Fig. 7, the device 10 may also be used to determine a parameter that is indicative of a water content of samples 12 of the plastic raw material.

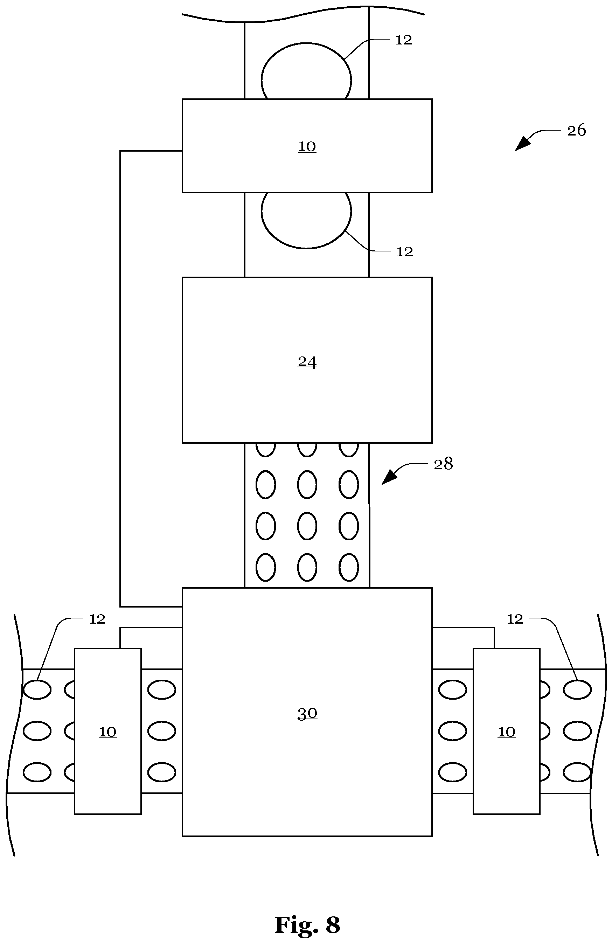

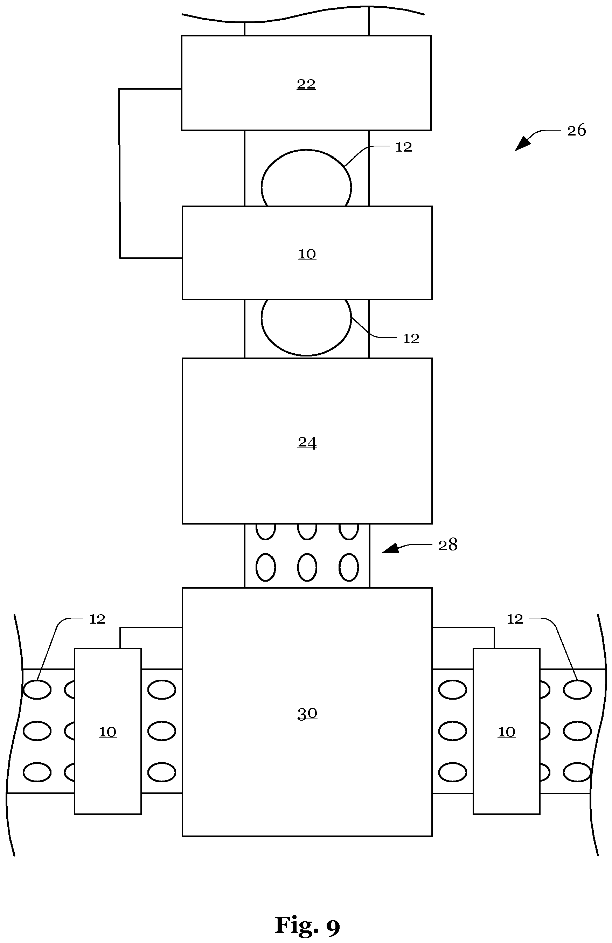

Furthermore, as shown in Fig. 8, in addition to determining a parameter that is indicative of a water content of the samples 12 of the plastic raw material, another device 10 may be arranged downstream of the apparatus 24 to determine a parameter that is indicative of a water content of samples 12 of the manufactured products. In addition, as shown in Fig. 9, a conditioner 22 may be arranged downstream of the device 10 to remove or add water to a sample 12 as required to make it conforming. Alternatively, or in addition, the conditioner 22 may take further measures to overcome adverse effects that would otherwise result from the non-conforming water content of the sample 12 as described above. Moreover, instead of a conditioner 22, a junction 20 which may be controlled by the controller 18 may be installed downstream of the device 10 to separate non-conforming samples 12 from conforming samples 12 as shown in Fig. 3.

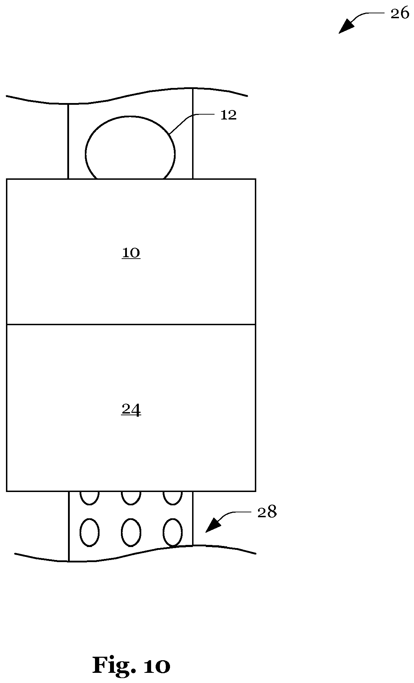

Fig. 10 schematically illustrates another example of integrating the device 10 into the system 26. There, the device 10 is positioned at the output of apparatus 24 where, for instance, physical parameters of the sample 12 such as the temperature and/or the rheology may undergo a transition. For instance, a thermoplastic from which the sample 12 is made or which the sample 12 comprises may cool and harden.

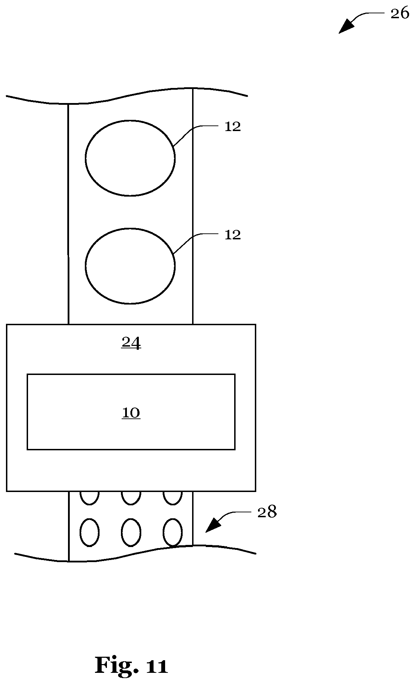

Fig. 11 schematically illustrates yet another example of integrating the device 10 into the system 26. There, the device 10 is integrated into the apparatus 24. For example, the apparatus 24 may be an extrusion apparatus and the device 10 may be integrated into the die of the extrusion apparatus.

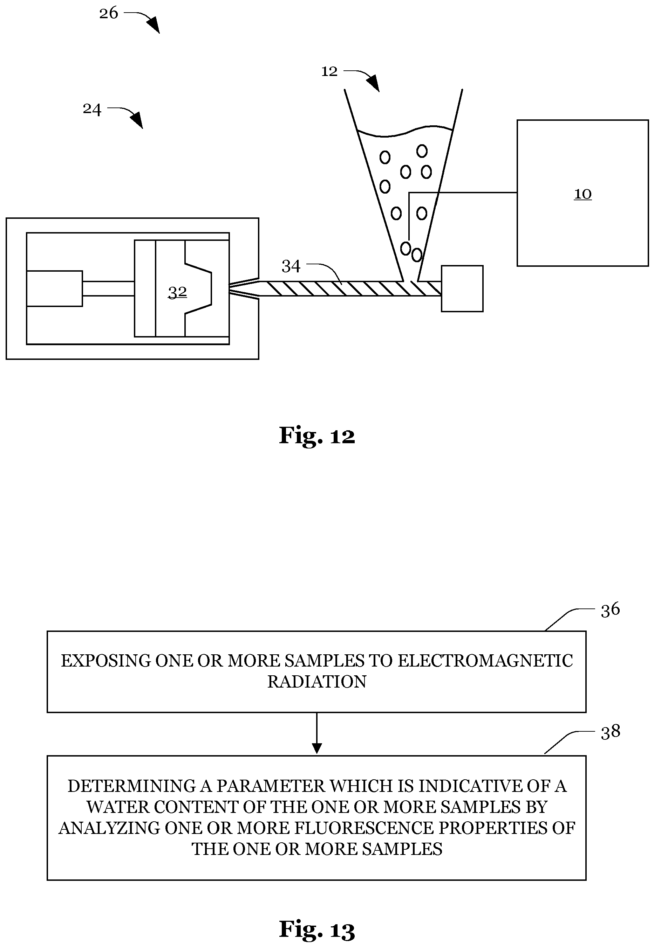

In another example shown in Fig. 12, the device 10 is used to measure the humidity of plastic raw material samples 12 with which an injection molding apparatus 24 is fed. The apparatus 24 comprises a mold cavity 32 into which molten raw material is injected by a reciprocating screw 34. The humidity of the plastic raw material samples 12 is used to control the process parameters in such a way as to achieve uniform product quality. As the viscosity of the material depends on its humidity, the injection of the material into the mold cavity 32 would vary if changes in humidity were not accounted for. However, by adapting one or more of injection speed, temperature, switch-over point, and holding pressure curve, a change in viscosity may be fully or at least partially compensated. The process parameters may be controlled using a closed control loop which may be based on a PID, MPC of fuzzy control strategy.

Fig. 13 shows a flow chart of steps which may be carried out by the device 10 or the system 26. At step 36, the method starts with exposing one or more samples 12 to electromagnetic radiation. At step 38, the method is continued with determining a parameter which is indicative of a water content of the one or more samples by analyzing one or more fluorescence properties of the one or more samples 12. The one or more fluorescence properties may include a fluorescence lifetime or a measure which is indicative of the fluorescence lifetime.

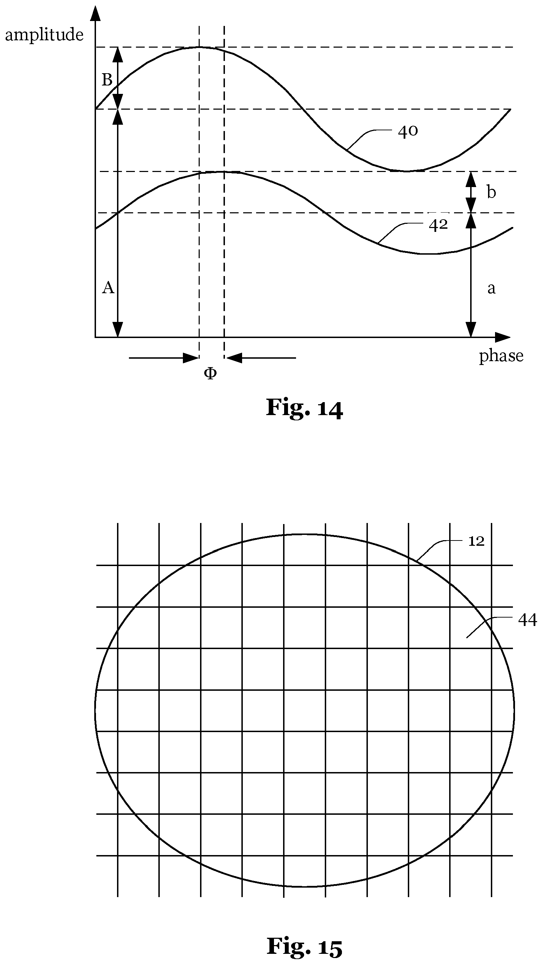

The fluorescence lifetime may be measured in the frequency domain as schematically illustrated in Fig. 14. As shown in Fig. 14, the signal 40 of the electromagnetic radiation incident on the samples 12 may be modulated causing fluorescent electromagnetic radiation having a signal 42 which is shifted in phase by $\Phi$.

$$\tau_P = \frac{\tan(\Phi)}{\omega}$$

may then be used as a measure of the fluorescence lifetime with $\omega$ being the angular frequency of the modulation. Notably, although a sinusoidal modulation is shown in Fig. 14, a rectangular modulation is also contemplated.

As schematically illustrated in Fig. 15, the method may comprise generating a two-dimensional representation of a sample 12, wherein the representation indicates a water content distribution across the sample 12. More specifically, the sample 12 may be divided into different parts 44 (or segments) and the water content of each part 44 may be determined individually. This may allow localizing aberrations which may be particularly helpful if the effect of a non-conforming water content differs from part 44 to part 44. For example, a product may have parts 44 that are particularly susceptible to strain where a non-conforming water content may have more disastrous effects than elsewhere.

REFERENCE SIGNS LIST

| 10 | device |
| 12 | sample |
| 14 | emitter |
| 16 | detector |
| 18 | controller |
| 20 | junction |
| 22 | conditioner |
| 24 | apparatus |
| 26 | system |
| 28 | raw material |
| 30 | mixer |
| 32 | mold cavity |
| 34 | screw |
| 36 | step |
| 38 | step |
| 40 | signal |
| 42 | signal |
| 44 | part |

## Claims

1. A method, comprising:

   exposing (36) one or more samples (12) to electromagnetic radiation; and
   determining (38) a parameter which is indicative of a water content of the one or more samples (12) by analyzing one or more fluorescence properties of the one or more samples (12).

2. The method of claim 1, wherein the one or more fluorescence properties include a fluorescence lifetime or a measure which is indicative of the fluorescence lifetime.

3. The method of claim 2, wherein the one or more fluorescence properties include a fluorescence intensity or a measure which is indicative of the fluorescence intensity.

4. The method of claim 2 or 3, wherein the one or more samples (12) comprise organic matter, preferably autofluorescent organic matter.

5. The method of claim 4, wherein the one or more samples (12) comprise plastic, preferably autofluorescent plastic.

6. The method of any one of claims 2 to 5, further comprising:
   controlling a manufacturing process which involves processing the one or more samples (12), based on the parameter.

7. The method of claim 6, wherein controlling the manufacturing process based on the parameter involves increasing or decreasing a water content of a raw material which comprises the one or more samples (12) if the parameter is outside a reference range.

8. The method of claim 6, wherein controlling the manufacturing process based on the parameter involves identifying a sample (12) or a product which comprises the one or more samples (12) as non-conforming if the parameter is outside a reference range.

9. The method of any one of claims 2 to 8, further comprising:
   collecting information which is indicative of a water content of different parts of one of the one or more samples (12) by analyzing the one or more fluorescence properties of the different parts.

10. The method of claim 9, further comprising:

    generating a two-dimensional or three-dimensional representation of said one of the one or more samples (12), wherein the representation indicates a water content distribution across said one of the one or more samples (12); and
    comparing the representation to a model or a reference.

11. A system (26), comprising:

    an apparatus (24) for manufacturing a product from plastic; and
    an inline monitoring device (10) which determines a parameter that is indicative of a water content of the plastic by analyzing one or more fluorescence properties of the plastic.

12. The system (26) of claim 11, wherein the inline monitoring device (10) is configured to measure a fluorescence lifetime of the plastic in a frequency domain.

13. The system (26) of claim 12, further comprising:
    a camera, wherein the inline monitoring device (10) is configured to record image data of the plastic as the plastic passes through an area onto which the camera is focused.

14. The system (26) of claim 12 or 13, wherein the system (26) is configured to switch a supply or to modify a pretreatment of the plastic if the parameter is outside a reference range.

15. The system (26) of any one of claims 12 to 14, further comprising:
    another inline monitoring device (10) which determines another parameter that is indicative of a water content of the product by analyzing one or more fluorescence properties of the product.

**Fig. 1**

**Fig. 2**

**Fig. 3**

**Fig. 4**

**Fig. 5**

**Fig. 6**

**Fig. 7**

**Fig. 8**

**Fig. 9**

**Fig. 10**

26

12

12

24

10

28

Fig. 11

**Fig. 12**

EXPOSING ONE OR MORE SAMPLES TO ELECTROMAGNETIC RADIATION

DETERMINING A PARAMETER WHICH IS INDICATIVE OF A WATER CONTENT OF THE ONE OR MORE SAMPLES BY ANALYZING ONE OR MORE FLUORESCENCE PROPERTIES OF THE ONE OR MORE SAMPLES

**Fig. 13**

**Fig. 14**

**Fig. 15**

Europäisches
Patentamt
European
Patent Office
Office européen
des brevets

# EUROPEAN SEARCH REPORT

**Application Number**

EP 23 20 7597

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/238801 A1 (CAMPBELL ERIC J [US] ET AL) 23 August 2018 (2018-08-23) | 1,3-11, 13-15 | INV. G01N33/44 |
| Y | * abstract; figures 1-4 * <br> * paragraph [0001] – paragraph [0035] * <br> ----- | 2,12 | G01N21/84 <br> G01N21/64 |
| Y | US 2023/168197 A1 (HAYAKAWA TOMOHIKO [JP] ET AL) 1 June 2023 (2023-06-01) <br> * abstract * <br> * paragraph [0057] – paragraph [0094] * <br> ----- | 2,12 | |

**TECHNICAL FIELDS SEARCHED (IPC)**

G01N

The present search report has been drawn up for all claims

| Place of search | Date of completion of the search | Examiner |
|---|---|---|
| Munich | 17 April 2024 | Pisani, Francesca |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 549 932 A1**

## ANNEX TO THE EUROPEAN SEARCH REPORT
## ON EUROPEAN PATENT APPLICATION NO.

EP 23 20 7597

17-04-2024

| Patent document cited in search report | | Publication date | Patent family member(s) | | | Publication date |
|---|---|---|---|---|---|---|
| US 2018238801 | A1 | 23-08-2018 | US | 2018238801 | A1 | 23-08-2018 |
| | | | US | 2019242821 | A1 | 08-08-2019 |
| US 2023168197 | A1 | 01-06-2023 | CN | 115298537 | A | 04-11-2022 |
| | | | JP | 7345939 | B2 | 19-09-2023 |
| | | | JP | WO2021187536 | A1 | 23-09-2021 |
| | | | US | 2023168197 | A1 | 01-06-2023 |
| | | | WO | 2021187536 | A1 | 23-09-2021 |

EPO FORM P0459

For more details about this annex : see Official Journal of the European Patent Office, No. 12/82